# EUROPÄISCHE PATENTANMELDUNG

(11) **EP 1 283 025 A1**
(43) Veröffentlichungstag der Anmeldung: **12.02.2003**
(21) Anmeldenummer: 01118821.6
(22) Anmeldetag: 10.08.2001
(51) Int. Cl.: A61B 17/12

(54) **Ballonokklusionsvorrichtung**

(71) Anmelder: Stöckert Instrumente GmbH, 80939 München (DE)
(72) Erfinder: Hahn, Andreas, Dr., 82335 Berg (DE); Göllner, Marcus, 80797 München (DE); Wetzig, Michael, 80339 München (DE); Bernhardt, Peter, Dr., 81825 München (DE)
(74) Vertreter: HOFFMANN - EITLE

(57) **Zusammenfassung**

Die beschriebene Ballonokklusionsvorrichtung umfasst eine Kanüle 1 und eine Okklusionseinrichtung 3, der eine Dilatationsflüssigkeit zuegführt wird. Im Inneren der Kanüle 1 ist ein Kernkörper 6 angeordnet, der einerseits das Volumen im Inneren der Kanüle 1 und andererseits die mechanische Flexibilität der Kanüle 1 verringert. Dadurch wird weniger Dilationsflüssigkeit benötigt und gleichzeitig die Handhabbarkeit der Vorrichtung verbessert.

## Beschreibung

Die Erfindung betrifft eine Ballonokklusionsvorrichtung insbesondere für den Einsatz in der Herzchirurgie zum Okkludieren der Aorta.

Ballonokklusionsvorrichtungn dieser Art sind bekannt und finden ihren Einsatz bei herzchirurgischen Einsätzen, bei denen die Aorta intraluminal blockiert werden muss. Die intraluminale Okkludierung der Aorta tritt anstelle der Abklemmung mit einer quer angesetzten Klemme, da bei dieser Art der Okkludierung neben Beschädigungen des Gefäßes auch die Gefahr besteht, dass sich mehr oder weniger große Partikel von der Wandung der Aorta lösen und in den Blutstrom des Patienten gelangen, was unter anderem zu Hirnembolien und die damit verbundenen neurologischen Ausfälle (Hirninfarkt) führen kann.

Ballonokklusionsvorrichtungen sind beispielsweise bekannt aus EP 1 086 717 A1 und DE 195 15 933 A1.

Aus der Sicht des mit der Ballonokklusionsvorrichtung arbeitenden Chirurgen steht eine einfache und sichere Handhabung der Okklusionsvorrichtung im Vordergrund, wobei insbesondere auf die Handhabbarkeit während des Einführens und Positionierens und auf eine schnelle Okkludierung Wert gelegt wird. Nachdem die Ballonokklusionsvorrichtung positioniert und die Aorta intraluminal okkludiert ist, muss die Kanüle ihre Position bei einem Druck von ca. 100 bis maximal 200 mmHg bzw. einem Fluss von 2 bis 6 l/min beibehalten. Dies bedeutet, dass zum Okkludieren vergleichsweise hohe Drücke erzeugt werden müssen.

Vor diesem Hintergrund besteht das von der Erfindung zu lösende Problem darin, eine Ballonokklusionsvorrichtung anzugeben, die beim Einführen und Positionieren in der Aorta einfacher zu handhaben ist und mit der die Okkludierung der Aorta möglichst schnell vorgenommen werden kann, ohne dass die Gefahr besteht, dass die Positionierung der Ballonokklusionsvorrichtung während des herzchirurgischen Eingriffs verloren geht.

Gelöst wird dieses Problem durch eine Ballonokklusionsvorrichtung mit den Merkmalen des Patentanspruchs 1. Vorteilhafte Ausgestaltungen ergeben sich aus den Unteransprüchen.

Im folgenden wird die Erfindung anhand eines Ausführungsbeispiels unter Bezugnahme auf die beigefügten Figuren genauer erläutert, in denen zeigt:
- Fig. 1: eine erfindungsgemäße Ballonokklusionsvorrichtung,
- Fig. 2: einen Querschnitt durch die erfindungsgemäße Ballonokklusionsvorrichtung an der Stelle A-A;
- Fig. 3: mehrere Querschnittsformen des Kernkörpers der erfindungsgemäßen Ballonokklusionsvorrichtung;
- Fig. 4: eine Querschnittsansicht einer weiteren Ausgestaltung des Kernkörpers der erfindungsgemäßen Ballonokklusionsvorrichtung;
- Fig. 5: eine Querschnittsansicht einer weiteren Ausgestaltung des Kernkörpers der erfindungsgemäßen Ballonokklusionsvorrichtung;
- Fig. 6: eine geschnittene Ansicht einer weiteren Ausgestaltung der Verschlusseinrichtung der erfindungsgemäßen Ballonokklusionsvorrichtung;
- Fig. 7: eine geschnittene Ansicht einer weiteren Ausgestaltung der Verschlusseinrichtung der erfindungsgemäßen Ballonokklusionsvorrichtung;
- Fig. 8: eine geschnittene Ansicht einer weiteren Ausgestaltung der Verschlusseinrichtung der erfindungsgemäßen Ballonokklusionsvorrichtung; und
- Fig. 9: eine geschnittene Ansicht einer weiteren Ausgestaltung der Verschlusseinrichtung und Sensoren im Kernkörper der erfindungsgemäßen Ballonokklusionsvorrichtung.

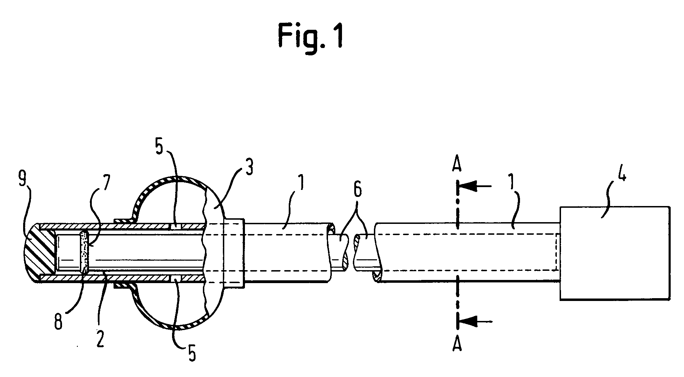

Wie in Fig. 1 gezeigt, besteht das hier beschriebene Ausführungsbeispiel der erfindungsgemäßen Ballonokklusionsvorrichtung aus einer langgestreckten flexiblen Kanüle 1, die im Inneren ein Lumen 2 umschließt und an deren distalem Ende eine Okkludierungseinrichtung, beispielsweise zumindest ein Okklusionsballon 3 angeordnet ist, der aus einem elastisch Kunststoff, beispielsweise Polyethylen besteht und der über eine ausreichende Formbeständigkeit und Steifigkeit verfügt, um nach dem Erweitern einen sicheren Abschluss der Aorta und eine sichere Positionierung der Ballonokklusionskanüle in der Aorta zu gewährleisten. Der Durchmesser des Okklusionsballons 3 ist an den Innendurchmesser der Aorta angepasst und liegt in der Größenordnung von 20 bis 30 mm.

Durch das Kanülenlumen 2 wird mit Hilfe einer geeigneten Zuführeinrichtung eine Dilatationsflüssigkeit, beispielsweise physiologische Kochsalzlösung dem Okklusionsballon 3 zugeführt, um ihn zu erweitern. Dazu ist am proximalen Ende der Ballonokklusionsvorrichtung ein Anschluss 4 für die Dilatationsflüssigkeit-Zuführungeinrichtung vorgesehen, bei der es sich beispielsweise um eine Glas- oder Kunststoff-Spritze handelt. Der Anschluss ist vorzugsweise nach dem bekannten "Luer Lock"-Prinzip gestaltet, der sich dadurch auszeichnet, dass durch Verbinden der Spritze mit dem Anschluss 4, beispielsweise durch Einstecken oder Eindrehen, eine Druckverbindung zum Lumen 2 der Kanüle 1 hergestellt und nach Entfernen der Spritze das Lumen 2 durch den Anschluss 4 druckerhaltend verschlossen wird. Die zugeführte Dilataionsflüssigkeit tritt aus einer oder mehreren Durchtrittsöffnungen 5 aus dem Lumen 2 der Kanüle 1 in den Okklusionsballon 3 aus und Erweitert diesen entsprechend der vom Benutzer zugeführten Menge an Dilatationsflüssigkeit.

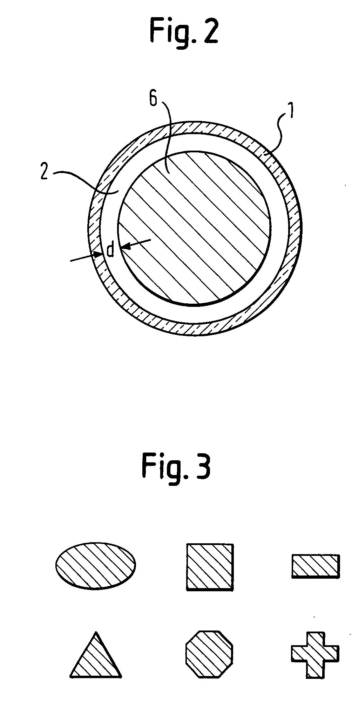

Erfindungsgemäß ist im Inneren der Kanüle 1 ein Kernkörper 6 vorgesehen, der sich bei dem hier gezeigten Ausführungsbeispiel über die gesamte Länge der Kanüle 1 erstreckt und das Kanülenlumen 2 zum Teil ausfüllt, indem er einen Teil, vorzugsweise 50% der Querschnittsfläche der Kanüle 1 derart einnimmt, dass nur der verbleibende Teil für die Zuführung der Dilatationsflüssigkeit zur Okklusionseinsrichtung 3 verfügbar ist. Wie in Fig. 2 erkennbar, reduziert der Kernkörper 5 das Volumen, das von der Dilatationsflüssigkeit in dem Kanülenlumen 2 zu füllen ist. Dies bedeutet, dass eine sehr viel kleinere Menge Dilatationsflüssigkeit ausreichend ist, um den Okklusionsballon 3 zu erweitern, da das Kanülenlumen 2 nun nicht mehr vollständig mit Dilatationsflüssigkeit aufgefüllt werden muss. Das noch verbleibende Volumen wird bestimmt durch den Spalt d, der sich um den Kernkörper 6 herum im Inneren der Kanüle 1 über deren Längserstreckung hinweg ergibt. Damit wird durch den erfindungsgemäßen Kernkörper 6 im Inneren der Kanüle 1 erreicht, dass mit einer kleineren Menge Dilatationsflüssigkeit und damit schneller eine Dilatation des Okklusionseinrichtung 3 und damit eine Okkludierung der Aorta erreicht werden kann. Für den Chirurg hat dies zur Folge, dass er mit einer sehr kurzen Betätigung der Fördervorrichtung für die Dilatationsflüssigkeit ein Okkludieren der Aorta und eine Positionsfixierung der Ballonokklusionsvorrichtung erreichen kann.

Erfindungsgemäß ist der Kernkörper 6 ist gleichzeitig so gestaltet, dass er aufgrund seiner mechanischen Eigenschaften den Chirurg bei der Handhabung der Ballonokklusionskanüle unterstützt. Die Form, insbesondere der Querschnitt, und das Material des Kernkörpers 6 sind entsprechend gewählt, so dass der Chirurg eine Unterstützung beim Einführen der erfindungsgemäßen Ballonokklusionsvorrichtung erfährt, ohne dass das Einführen durch eine zu geringe Flexibilität behindert wird. Regelmäßig führt das zu einer Verringerung der Kanülenlumens 2. Dies wird durch geeignete Auswahl des Materials sowie durch eine angepasste Gestaltung der Form und des Querschnitts, insbesondere der Querschnittgröße erreicht. Im einfachsten Fall handelt es sich bei dem Kernkörper 6 um einen massiven Stab aus einem flexiblen und für medizinische Anwendungen geeigneten Material, beispielsweise Teflon, der in dem Kanülenlumen 2 angeordnet ist. Der Kernkörper kann eine Armierung aus einem formbaren Metall aufweisen oder aus einem formbaren Material, beispielsweise einem geeigneten Metall bestehen, beispielsweise Nitinol, so dass die erfindungsgemäße Kanüle 1 auf Grund der Formbarkeit des Kernkörpers 6 vor dem Einführen in eine gewünschte Form gebracht werden kann, die die Kanüle dann aufgrund der Formbarkeit des Kernkörpers 6 im wesentlichen beibehält. Die Verformung kann jederzeit rückgängig gemacht werden, wobei durch geeignete Wahl des Materials die Rückverformung in die Ausgangsform unterstützt werden kann. Vorzugsweise besitzt der Kernkörper 6 einen kreisförmigen Querschnitt und ist damit an den Querschnitt der Kanüle 1 angepasst. Der Kernkörper 6 kann auch die in Fig. 3 gezeigten Querschnitte aufweisen, also elliptisch, quadratisch, rechteckig, dreieckig, mehreckig oder kreuzförmig sein.

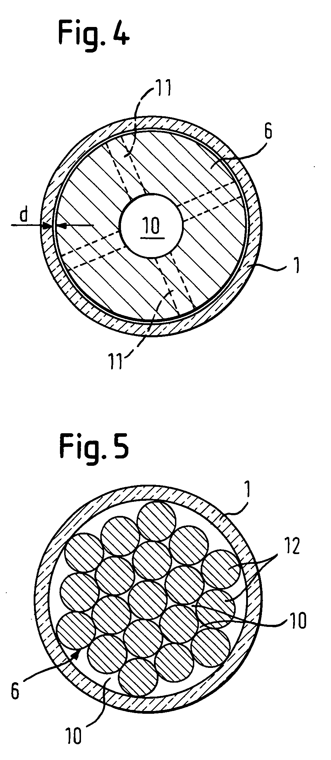

In Fig. 4 ist eine weitere Ausgestaltung des Kernkörpers 6 gezeigt, bei der der Kernkörper 6 einen Aussendurchmesser aufweist, der im wesentlichen dem Innendurchmesser der Kanüle 1 entspricht. Damit Dilatationsflüssigkeit durch die Kanüle 1 gefördert werden kann, besitzt der Kernkörper 6 ein Kernkörperlumen 10, das sich vom proximalen Ende bis in den Bereich des distalen Endes des Kernkörpers 6 erstreckt, so dass die mit Hilfe der Zuführeinrichtung zugeführte Dilatationsflüssigkeit durch das Kernkörperlumen 10 strömen kann. Der Spalt d zwischen Kernkörper 6 und Kanüle 1 ist klein und kann im Grenzfall sogar ganz vermieden werden.

Bei dem in Fig. 4 gezeigten Ausführungsbeispiel besitzt das Kernkörperlumen 10 einen kreisförmigen Querschnitt, jedoch können auch hier andere Querschnitte vorgesehen sein. Außerdem kann der Kernkörper 6 mehr als ein Kernkörperlumen 10 aufweisen.

Damit die Dilatationsflüssigkeit aus dem Kernkörperlumen 10 austreten und in den Okklusionsballon 3 gelangen kann, besitzt der Kernkörper 6 zumindest im Bereich seines distalen Endes Verbindungsöffnungen 11, die in Fig. 4 gestrichelt dargestellt sind und die das bzw. die Kernkörperlumen 10 mit der Oberfläche ihres Kernkörpers 6 und damit dem Kanülenlumen 2 oder unmittelbar mit den Durchtrittsöffnungen 5 der Kanüle 1 verbinden. Die Dilatationsflüssigkeit gelangt dann aus dem bzw. den Kernkörperlumen 10 durch die Verbindungsöffnungen 11 in das Kanülenlumen 2 und von dort oder direkt von den Verbindungsöffnungen 11 durch die Durchtrittsöffnungen 5 in den Okklusonsballon 2. Die Anzahl, die Größe und der Querschnitt der Verbindungsöffnungen 11 sind in weiten Bereichen frei wählbar und nach den Gegebenheiten der Kanüle 1 und des Okklusionsballons 2 zu gestalten.

Wie in Fig. 5 gezeigt,kann der Kernkörper 6 auch in Form eines Bündels einzelner oder miteinander verbundener Fasern 12 ausgebildet werden. Dabei kann auch das Faserbündel 12 einen Gesamtquerschnitt aufweisen, der im wesentlichen dem Innendurchmesser der Kanüle 1 entspricht, so dass dann die zwischen den Fasern 12 vorhandenen Zwischenräume als Kernkörperlumen 10 anzusehen sind, wie in Fig. 5 gezeigt. Das Faserbündel 12 kann aber auch als Kernkörper 6 so gestaltet sein, dass ein Spalt d zur Kanüle frei bleibt, wie in Fig. 2 für einen massiven Kernkörper gezeigt. Die Dilatationsflüssigkeit strömt durch die Zwischenräume 10 des Faserbündels 12 und gelangt so in das Kanülenlumen 2 und durch die Durchtrittsöffnungen 5 in den Okklusionsballon 3. In einer weiteren Ausgestaltung kann der Kernkörper auch aus einem porösen Material bestehen, so dass ähnlich dem Faserbündel ein Kernkörper mit zahlreichen Kernkörperlumen vorgesehen ist.

Wie in Fig. 1 gezeigt, ist in einer weiteren Ausgestaltung bei der erfindungsgemäßen Ballonokklusionsvorrichtung eine Fixierungseinrichtung am distalen Ende des Kernkörpers 6 vorgesehen, die den Kernkörper 6 beim distalen Ende in der Kanüle 1 festlegt, d.h eine Lagefixierung des Kernkörpers 6 am distalen Ende bewirkt. Dazu besitzt der Kernkörper 6 eine umlaufende Nut 7, um darin einen Fixierring 8 aufzunehmen. Dadurch kann die erfindungsgemäße Ballonokklusionsvorrichtung am distalen Ende mit Hilfe des Kernkörpers 6 und des Fixierrings 8 aber bereits so sicher verschlossen, dass ein Austreten der Dilatationsflüssigkeit am distalen Ende sicher vermieden wird. Das bedeutet, dass der Fixiereinrichtung zusätzliche eine Abdichtfunktion zukommen kann. Durch die Gestaltung, insbesondere die Größe und das Material des Fixierrings 8 in Abstimmung mit dem Durchmesser des Kernkörpers 6 und des Innendurchmessers der Kanüle 1 kann nicht nur eine sichere Fixierung sondern auch ein sicherer Verschluss der Ballonokklusionsvorrichtung am distalen Ende selbst bei den hier in Rede stehenden Drücken von bis zu etwa 0,5 bar.

Vorzugsweise ist am distalen Ende der erfindungsgemäßen Ballonokklusionsvorrichtung jedoch zusätzlich eine Verschlusseinrichtung 9 des Kanülenlumens 2 vorgesehen, so dass neben dem Fixierring 7 am Kernkörper 6 noch ein Verschluss 9 ein Austreten der Dilatationsflüssigkeit aus dem Kanülenlumen 2 verhindert. Bei der Verschlusseinrichtung kann es sich um den in Fig. 1 gezeigten Stopfen 9 handeln.

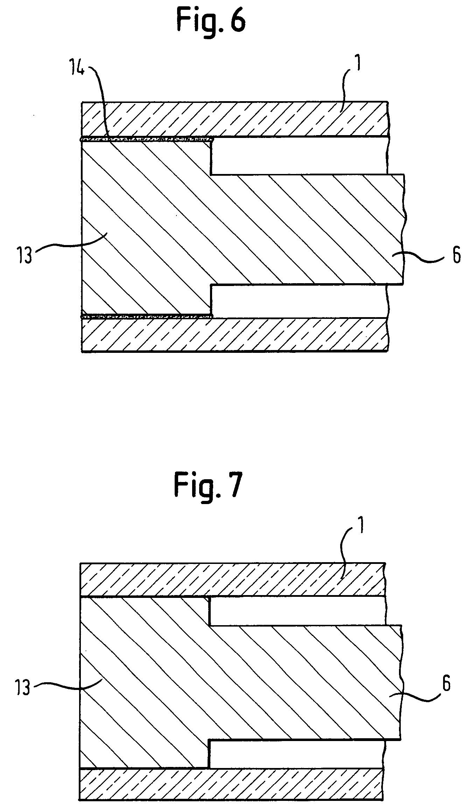

Wie in den Fig. 6 bis 9 gezeigt, kann auch der Kernkörper 6 am distalen Ende als Verschlusseinrichtung 9 ausgebildet werden.

In einer ersten Ausgestaltung, gemäß Fig.6, ist dazu der Durchmesser des Kernkörpers 6 zumindest im Bereich 13 des distalen Endes so gewählt, dass durch Verkleben mittels einer Klebstoffschicht 14 das distale Ende des Kernkörpers 6 mit der Kanüle 1 verbunden wird, wodurch die Kanüle 1 am distale Ende verschlossen wird.

In einer zweiten Ausgestaltung, gemäß Fig. 7, kann ein Bereich 13 am distalen Ende des Kernkörpers 6 so ausgebildet sein, dass die äußere Oberfläche des Kernkörpers 6 in diesem Endbereich 13 an der inneren Oberfläche der Kanüle 1 anliegt und ein Verschluss des distalen Endes der Kanüle 1 erfolgt.

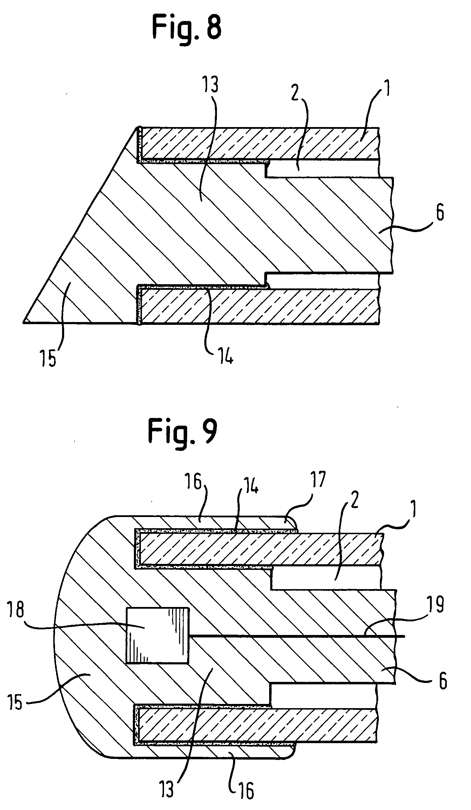

Ferner kann in einer weiteren Ausgestaltung, gemäß Fig. 8, das distale Ende des Kernkörpers 6 aus der Kanüle herausragen, wobei der herausragende Bereich 15 einen Durchmesser besitzt, der vorzugsweise größer ist als der Innendurchmesser der Kanüle 1. Der herausragende Bereich 15 des Kernkörpers ist vorzugsweise zur Unterstützung des Einführens der erfindungsgemäßen Kanüle 1 ausgebildet. Zum Beispiel besitzt das herausragende Bereich 15 des Kernkörpers 6 eine abgerundete oder, wie ind Fig. 8 gezeigt, eine zugespitzte, vorzugsweise eine schräg zugespitzte Form. In Fig.8 ist ferner eine Ausgestaltung gezeigt, bei der am distalen Ende ein Verklebungsbereich 13 am Kernkörper 6 vorgesehen und der Kernkörper in diesem Bereich 13 und im Bereich der Stirnkanten der Kanüle 1 mittels Kleber 14 mit der Kanüle 1 verklebt ist.

Schließlich kann, wie in Fig. 9 gezeigt, der herausragende Bereich 15 des Kernkörpers 6 ein Wandelements 16 aufweisen, das sich vom distalen Ende der Kanüle 1 in Richtung auf das proximale Ende der Kanüle 1 erstreckt und dadurch einen Kragen bildet, der außen um die Kanüle 1 herum an deren distalem Ende ausgebildet ist. Der dem proximalen Ende der Kanüle zugewandte Bereich 17 des Wandelements 16 ist abgerundet, um ein Rückziehen der Kanüle 1 aus der Aorta nicht zu behindern. Der Kragen 16 des Kernkörpers 6 ragt vorzugsweise bis in den Bereich des Fixierrings 8 zurück. Dadurch wird verhindert, dass sich die Kanüle 1 aufgrund des Innendrucks im Bereich des Ringes 8 erweitert und so die Fixierfunktion des Fixierringes 8 verloren geht.

An dem Kernkörper können ein oder mehrere Sensoren, auch Ultra-Schall-Sensoren oder optische Sensoren angebracht sein, wie in Fig. 9 durch das Bezugszeichen 18 angedeutet ist, die unterschiedliche physiologische Parameter erfassen, wie beispielsweise Druck, Temperatur, Sauerstoffpartialdruck etc. Der Kernkörper 6 dient dann als Träger dieser Sensoren und vorzugsweise auch als Träger der Sensorverbindungsleitungen 19 , über die die Sensorsignale zum distalen Ende und aus der erfindungsgemäßen Kanüle 1 heraus geführt werden. Der bzw. die Sensoren 18 können an bzw. in dem Kernkörper 6 im Bereich der Kanüle 1 und/oder an dem aus der Kanüle herausragenden Bereich 15 angeordnet sein.

Der bei einer erfindungsgemäßen Kanüle vorgesehene Kerkörper 6 eignet sich auch zum Anbringen von Markierungen, durch die der Benutzer feststellen kann, wie weit das distale Ende der Kanüle 1 eingeführt wurde. Dazu ist es erforderlich, dass die Kanüle 1 aus einem durchsichtigen, zumindest aber transparenten Material besteht.

## Patentansprüche

1. Ballonokklusionvorrichtung mit
a. einer flexiblen Kanüle (1) mit Durchtrittsöfnungen (5) am distalen Ende;
b. einer Okklusionseinrichtung (3), die am distalen Ende der Kanüle (1) im Bereich der Durchtrittsöffnungen (5) angebracht ist;
c. einer Anschlusseinrichtung (4) für eine Zuführeinrichtung zum Zuführen einer Dilataionsflüssigkeit, die über die Kanüle (1) und die Durchtrittsöffnungen (5) in das Innere der Okklusionseinrichtung (3) zuführbar ist; und
d. einem Kernkörper (6), der im Inneren der Kanüle (1) angeordnet ist und der einerseits das Volumen im Inneren der Kanüle (1) und andererseits die mechanische Flexibilität der Kanüle (1) verringert.

2. Ballonokklusionsvorrichtung nach Anspruch 1, **dadurch gekennzeichnet, dass** sich der Kernkörper (6) im wesentlichen über die gesamte Länge der Kanüle (1) erstreckt.

3. Ballonokklusionsvorrichtung nach Anspruch 1 oder 2, **dadurch gekennzeichnet, dass** der Kernkörper (6) einen kreisförmigen, rechteckigen, quadratischen, mehreckigen oder kreuzförmigen Querschnitt besitzt.

4. Ballonokklusionsvorrichtung nach einem der Ansprüche 1 bis 3, **dadurch gekennzeichnet, dass** der Kernkörper (6) mindestens 50 % der Querschnittsfläche der Kanüle (1) ausfüllt.

5. Ballonokklusionsvorrichtung nach einem der Ansprüche 1 bis 4, **dadurch gekennzeichnet, dass** der Kernkörper am distalen Ende der Kanüle festgelegt ist.

6. Ballonokklusionsvorrichtung nach einem der Ansprüche 1 bis 5, **dadurch gekennzeichnet, dass** am distalen Ende des Kernkörpers (6) eine Fixiereinrichtung (7, 8) vorgesehen ist.

7. Ballonokklusionsvorrichtung nach Anspruche 5, **dadurch gekennzeichnet, dass** die Abdichtungseinrichtung als Fixierring (8) ausgebildet ist und der Kernkörper (6) eine Nut (7) aufweist, in der der Fixierring (8) angeordnet ist.

8. Ballonokklsionsvorrichtung nach einem der Ansprüche 5, 6 oder 7, **dadurch gekennzeichnet, dass** die Fixiereinrichtung (7, 8) die Kanüle (1) am distalen Ende abdichtet.

9. Ballonokklsionsvorrichtung nach einem der vorangegangenen Ansprüche, **dadurch gekennzeichnet, dass** der Kernkörper zumindest ein Kernkörperlumen (10) aufweist.

10. Ballonokklsionsvorrichtung nach Anspruch 9, **dadurch gekennzeichnet, dass** der Kernkörper (6) Verbindungsöffnungen (11) zwischen dem Kernkörperlumen (10) und dem Kanülenlumen bzw. den Durchtrittsöffnungen (5) der Kanüle (1) aufweist.

11. Ballonokklsionsvorrichtung nach einem der Ansprüche 1 bis 10, **dadurch gekennzeichnet, dass** der Kernkörper (6) massiv ausgebildet ist.

12. Ballonokklsionsvorrichtung nach einem der Ansprüche 1 bis 10, **dadurch gekennzeichnet, dass** der Kernkörper (6) als Faserbündel (12) ausgebildet ist.

13. Ballonokklsionsvorrichtung nach einem der Ansprüche 1 bis 10, **dadurch gekennzeichnet, dass** der Kernkörper (6) aus einem porösen Material besteht.

14. Ballonokklusionsvorrichtung nach einem der vorangegangenen Ansprüche, **dadurch gekennzeichnet, dass** der Kernkörper (6) aus einem formbaren Material, insbesondere einem seine Ausgangsform wiedereinnehmenden Material besteht.

15. Ballonokklusionsvorrichtung nach einem der vorangegangenen Ansprüche, **dadurch gekennzeichnet, dass** am distalen Ende der Kanüle (1) eine Verschlussrinrichtung (9) vorgesehen ist.

16. Ballonokklusionsvorrichtung nach Anspruch 14, **dadurch gekennzeichnet, dass** die Verschlusseinrichtung ein Stopfen (9) ist, der in dem distalen Ende der Kanüle (1) angeordnet ist.

17. Ballonokklsionsvorrichtung nach Anspruch 14, **dadurch gekennzeichnet, dass** der Kernkörper (6) einen Verschlussbereich (13) am distalen Ende aufweist.

18. Ballonokklsionsvorrichtung nach Anspruch 16, **dadurch gekennzeichnet, dass** der Verschlussbereich (13) des Kernkörpers (6) mit der Kanüle (1) verklebt ist.

19. Ballonokklusionsvorrichtung nach einem der Ansprüche 17 oder 18, **dadurch gekennzeichnet, dass** der Kernkörper (6) einen aus dem distalen Ende der Kanüle (1) herausragenden Bereich (15) aufweist.

20. Ballonokklsionsvorrichtung nach Anspruch 19, **dadurch gekennzeichnet, dass** der herausragende Bereich (15) des Kernkörpers (6) abgerundet oder zugespitzt, vorzugsweise schräg-zugespitzt ist.

21. Ballonokklusionsvorrichtung nach einem der Ansprüche 19 oder 20, **dadurch gekennzeichnet, dass** der Kernkörper (6) an der herausragenden Bereich (15) ein Wandelement (16) aufweist, das sich außen an der Kanüle (1) von deren distalen Ende in Richtung auf das proximale Ende der Kanüle (1) erstreckt.

22. Ballonokklusionsvorrichtung nach Anspruch 21, **dadurch gekennzeichnet, dass** das Wandelement (16) des Kernkörpers (6) umlaufend ausgebildet ist.

23. Ballonokklusionsvorrichtung nach einem der Ansprüche 21 oder 22, **dadurch gekennzeichnet, dass** das Wandelement (16) des Kernkörpers (6) an dem in proximaler Richtung angeordneten Bereich (17) abgerundet ausgebildet ist.

24. Ballonokklusionsvorrichtung nach einem der Ansprüche 21, 22 oder 23, **dadurch gekennzeichnet, dass** das Wandelement (16) sich bis in den Bereich des Fixierringes (8) erstreckt.

25. Ballonokklusionsvorrichtung nach einem der vorangegangenen Ansprüche, **dadurch gekennzeichnet, dass** an dem Kernkörper (6) Markierungen zur Unterstützung der Positionierung der Kanüle vorgesehen sind.

26. Ballonokklusionsvorrichtung nach einem der vorangegangenen Ansprüche, **dadurch gekennzeichnet, dass** in oder an dem Kernkörper (6) zumindest eine Sensoreinrichtung (18) für physiologische Parameter vorgesehen ist.

27. Ballonokklusionsvorrichtung nach Anspruche 25, **dadurch gekennzeichnet, dass** in oder an dem Kernkörper (6) Verbindungsleitungen (19) für die zumindest eine Sensoreinrichtung (18) vorgesehen sind.

28. Ballonokklusionsvorrichtung nach einem der vorangegangenen Ansprüche, **dadurch gekennzeichnet, dass** die Okklusionsvorrichtung als flexibler Ballon (3), vorzugsweise aus Kunststoff ausgebildet ist.
